# EUROPEAN PATENT APPLICATION

(11) **EP 2 716 241 A1**
(43) Date of publication of application: **09.04.2014**
(21) Application number: 13275208.0
(22) Date of filing: 11.09.2013
(51) Int. Cl.: A61B 17/3207, A61M 25/10

(54) **Scoring balloon and apparatus therefor**

(30) Priority: 05.10.2012 GB 201217865
(71) Applicant: Cook Medical Technologies LLC, Bloomington, IN 47404 (US)
(72) Inventor: Burton, David, Bloomington, IN 47408 (US); Elgaard, Per, 4690 Haslev (DK)
(74) Representative: Jehan, Robert

(57) **Abstract**

A scoring balloon (26) is provided with a plurality of scoring elements (34) and is wrapped onto a balloon catheter (12) such that the scoring elements (34) lie underneath the balloon wall (36) with the balloon wall (36) folded over the scoring elements (34). The scoring elements (34) are rounded at their extremities. The balloon (26) is folded onto the balloon catheter (12) in a tightly wrapped configuration.

## Description

### Technical Field

The present invention relates to a scoring balloon and to apparatus for and a method of pleating and wrapping such a balloon.

### Background of the Invention

Cutting or scoring balloons are known for opening occluded or constricted vessels of a patient, caused for instance by stenosis. Such balloons may have one or more blades or scoring elements fixed to or otherwise integral with the balloon wall, used for cutting or fragmenting stenosed material from the vessel wall. These balloons are generally effective in opening stenosed vessels. However, difficulties have generally arisen with the deployment of such balloons as a result of the perceived risk of the balloon wall being cut or torn by the cutting or scoring elements and of the cutting or scoring elements being exposed during the delivery process. As a result, these balloons tend to be wrapped in such a manner that the balloon wall is kept away from the cutting or scoring elements when the balloon is in a deflated state with the cutting or scoring elements inaccessible until the balloon has been inflated. This may be by a particular balloon folding arrangement or by provision of a protective element around the cutting or scoring elements. Whilst these methods may be effective in reducing the risk of damage to the balloon wall, they lead to a balloon which is loosely wrapped and which thus has a much greater deflated footprint than simple medical balloons, which are tightly wrapped onto the balloon catheter. A wider introducer assembly is harder to deploy endoluminally in a patient. Moreover, a balloon which has a greater deflated diameter is not suitable to treating heavily stenosed vessels, that is having only a small opening through the stenosed area, or for treating smaller diameter vessels.

Examples of prior art cutting or scoring balloons can be found for instance in US-7,413,558 and US-5,209,799. Apparatus for pleating or wrapping standard balloon catheters can be seen, for example, in US-7,762,804, US-7,407,377, US-7,618,252 and US-2005/0251194.

### Summary of the Invention

The present invention seeks to provide an improved scoring balloon and improved apparatus for and method of pleating a scoring balloon.

According to an aspect of the present invention, there is provided a pleated scoring balloon, the balloon including a flexible balloon wall having inner and outer surfaces, the balloon wall providing a balloon body portion extending along a longitudinal axis of the balloon and having a circumferential periphery; and a plurality of scoring elements on or attached to the outer surface of the balloon wall, said scoring elements extending substantially in the direction of said longitudinal axis and being spaced from one another along the circumferential periphery of the body portion, each scoring element exhibiting a rounded or compliant extremity; wherein balloon wall located between the scoring elements overlies and is in contact with the scoring elements.

The extremities of the scoring elements are preferably rounded to a radius of at least 0.025 millimetres. Such rounding ensures that the scoring elements do not cut the balloon wall. It has been found that scoring elements of such a nature can be as effective and in some instances more effective than sharp cutting elements in the form of blades. In most instances stenosis material can be removed by scoring rather than cutting the material, and that scoring reduces the risk of damage to the vessel wall.

Preferably, the scoring elements have their extremities rounded to a radius of around 0.025 millimetres to around 0.25 millimetres. One example of balloon formed of Nylon 12 ™ material has scoring elements rounded to a radius of around 0.05 millimetres.

It is to be understood that the scoring elements may be made of a compliant material, in which the extremities of the scoring elements partially flatten or become rounded upon application of pressure thereto, such as not to provide a sharp and hard cutting blade. Flattening of this nature could round the extremities to a radius of at least 0.025 millimetres as disclosed above. Thus, the scoring elements could have sharp extremities when not pressed but in practice do exhibit rounded extremities once pressure is applied to them, thereby avoiding risk of them cutting the balloon wall.

The skilled person will appreciate that the degree of rounding of the extremities of the scoring elements will be dependent upon the size of the balloon, the size of the scoring elements and the material used. What is relevant is that the scoring elements are either sufficiently rounded or sufficiently compliant not to cut into the balloon wall when the latter is wrapped over the scoring elements for delivery.

In the preferred embodiment, the wrapped scoring balloon has minimal airspaces. In particular, the wrapped balloon wall is in direct contact with, that is touches, the scoring elements over which it is wrapped.

The pleating of the balloon wall is advantageously in the same direction, that is clockwise or anti-clockwise.

In an embodiment, the scoring elements are made of a polymeric or elastomeric material. Advantageously, the scoring elements are unitary with the balloon wall, that is in the form of a single component. For this purpose, the scoring elements could constitute thickened portions of balloon wall in the form of ribs or ridges.

Advantageously, the scoring elements are made of the same material as the balloon wall.

In another embodiment, the scoring elements may be fixed or bonded to the balloon wall, for example by adhesive, weld or bond connections for instance. In this embodiment, the scoring elements could be in the form of a wire or flexible rod attached to the balloon wall so as to be integral therewith. The scoring elements are attached to the balloon wall along their entire lengths.

In other words, in the pleated balloon structure taught herein the scoring elements lie underneath the pleated balloon wall.

According to another aspect of the present invention, there is provided an introducer assembly including a balloon catheter having a proximal and a distal end and a scoring balloon at the distal end thereof, the scoring balloon being pleated and wrapped on the balloon catheter; the balloon including a flexible balloon wall having inner and outer surfaces, the balloon wall providing a balloon body portion extending along a longitudinal axis of the balloon and having a circumferential periphery; and a plurality of scoring elements on or attached to the outer surface of the balloon wall, said scoring elements extending substantially in the direction of said longitudinal axis and being spaced from one another along the circumferential periphery of the body portion, each scoring element having a rounded or compliant extremity; wherein balloon wall located between the scoring elements overlies and is in contact with the scoring elements.

Such a balloon arrangement represents a departure from the art, which in some cases taught a structure to maintain balloon wall material away from cutting or scoring elements on the balloon to prevent damage to the balloon wall. Such structures lead to an inability to wrap the balloon tightly to the catheter and thus require a substantially larger diameter catheter for delivery. A tighter wrapping also enhances trackability and pushability of the introducer assembly used for deploying the balloon.

Other art has taught providing a scoring wire which is detached from the balloon wall but this is considered to cause loss of scoring efficiency.

According to another aspect of the present invention, there is proved a method of pleating a scoring balloon, the balloon including a flexible balloon wall having inner and outer surfaces, the balloon wall providing a balloon body portion extending along a longitudinal axis of the balloon and having a circumferential periphery; and a plurality of scoring elements on or attached to the outer surface of the balloon wall, said scoring elements extending substantially along said longitudinal axis and being spaced from one another along the circumferential periphery of the body portion; the method including the steps of pleating balloon wall located between the scoring elements so as to overlie the scoring elements and wrapping the balloon wall tightly over the scoring elements.

### Brief Description of the Drawings

Embodiments of the present invention are described below, by way of example only, with reference to and as illustrated in the accompanying drawings, in which:
Figure 1 is a schematic diagram of an example of scoring balloon catheter assembly;
Figure 2 is a transverse cross-sectional view of a preferred embodiment of folded scoring balloon;
Figure 3 is a transverse cross-sectional view of the scoring balloon of Figure 2 after having been pleated by pleating apparatus;
Figure 4 is a view of part of a preferred embodiment of pleating apparatus for pleating a balloon as shown in Figure 3;
Figure 5 is a view of the folding apparatus of Figure 4 showing a scoring balloon being positioned inside the pleating blades of the apparatus;
Figure 6 shows the apparatus of Figure 5 with the blades closing in on the balloon so as to pleat the balloon walls;
Figure 7 shows the blades in a fully closed position with the balloon folded; and
Figures 8 and 9 show an embodiment of wrapping apparatus for wrapping the pleated balloon tightly onto its carrier catheter.

### Description of the Preferred Embodiments

It is to be understood that the drawings are schematic and are not to scale.

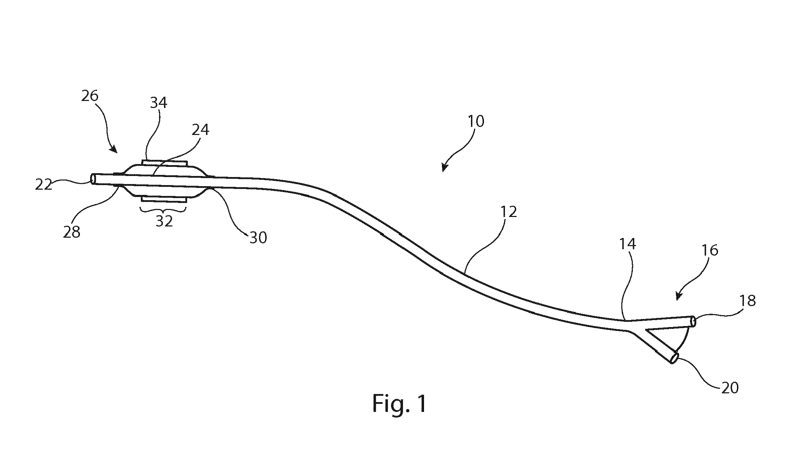

Referring to Figure 1, there is shown in schematic form an example of scoring balloon catheter assembly 10 which has, in the inflated state shown in Figure 1, a generally conventional structure. More specifically, the assembly 10 includes a catheter 12 having a proximal end 14 coupled to a fitting 16, which in this example is a Y-fitting. The fitting 16 includes a first port 18 for the passage of a guide wire and a second port 20 for feeding inflation fluid into the catheter 12. The catheter 12 typically has at least two lumens passing therethrough, one from the first port 18 all the way to the distal end 22 of the catheter 12, for receiving a guide wire. The second lumen extends from the port 20 to an opening 24 proximate the distal end 22 of the catheter.

Attached to the distal end of the catheter is a scoring balloon 26, which at its ends 28 and 30 is fixed to the catheter 12 in fluid-tight manner. The balloon 26 also includes a generally cylindrical body portion 32, which has attached thereto or otherwise integral herewith a plurality of scoring elements 34 which extend generally in a longitudinal direction of the balloon 26, that is parallel the axis of the catheter 12 and of the body portion of the balloon. As will be apparent from Figure 1, the opening 24 extends into the internal chamber of the balloon 26, thereby providing for the passage of inflation fluid into the balloon in order to inflate this.

Figure 1 shows the balloon 26 in its typically inflated form.

The balloon 26 is generally made of a thin-walled material, typically formed of one or more layers and may be compliant or non-compliant. A compliant balloon will continue to expand as the pressure of inflation fluid increases, whereas a non-compliant balloon will maintain a substantially constant inflated diameter over a range of operating pressures. The balloon wall may include one more strengthening elements such as strengthening wires or sleeves, preferably embedded within the thickness of the balloon wall.

The scoring elements 34 extend along the longitudinal axis of the balloon 26 but in other embodiments may extend at an angle to this, for example by being generally helically disposed.

The elements 34 providing a scoring function, and may be formed as relatively blunt ribs extending along and radially out of the balloon wall. These ribs may be made of a variety of materials including metal or metal alloy, polymer, and in preferred embodiments of the same material as that of the balloon wall. The scoring elements 34 provide relatively rigid members which act to score or scrape stenosed material away from a vessel wall.

For insertion into a patient's vasculature, the balloon 26 is deflated, pleated and wrapped around the catheter 12, and delivered through an introducer assembly. In the prior art, cutting or scoring balloons are typically wrapped loosely in an attempt to protect the balloon wall from the cutting or scoring elements and also in order to conceal the cutting or scoring elements until the balloon has been inflated. This, however, results in cutting or scoring balloons which are only loosely wrapped on the balloon catheter and which thus have a much greater delivery diameter. The teachings herein propose a different pleating and wrapping arrangement.

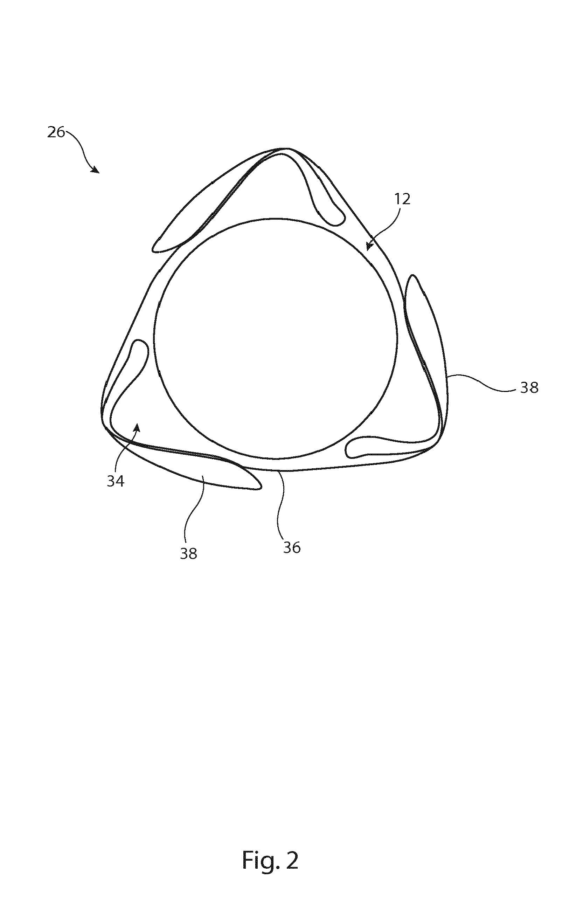

Specifically, referring to Figure 2, the balloon 26 of the preferred embodiment is shown in a wrapped configuration around the catheter 12, as it is configured for delivery through an introducer sheath or catheter of an introducer assembly. The balloon 26 includes a balloon wall 36 made of flexible material and which, when inflated, has a much larger diameter than the diameter of the catheter 12. As can be seen in Figure 2, the balloon wall is pleated and wrapped in such a manner that sections 38 of the balloon wall are pleated and wrapped over the scoring elements 34 and in contact with the scoring elements 34 when the balloon is in its wrapped state.

As can be seen in Figure 2, the scoring balloon 26 is tightly wrapped onto the catheter 12, with preferably minimal space between the overlying balloon wall and the underlying scoring elements. This is a much tighter wrapped balloon compared to prior art arrangements.

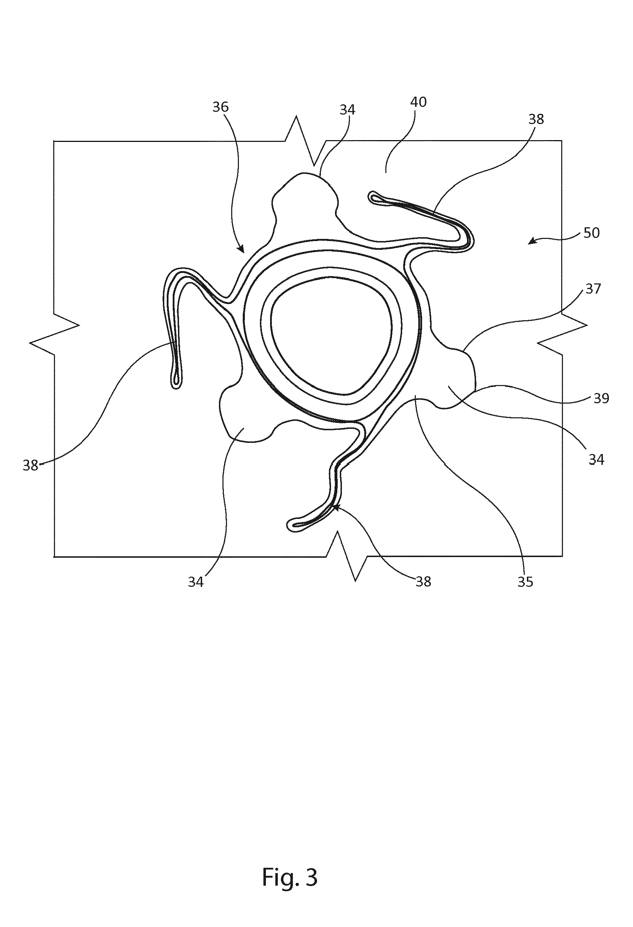

Figure 3 is a photograph of an actual example of scoring balloon in transverse cross-section. The balloon 50 is shown following pleating of the balloon by means of pleating apparatus described below and before it has been tightly wrapped by a wrapping device. Figure 3 shows in clearer detail the pleated balloon wall 36, creating pleated regions 38 which are pleated and subsequently wrapped over the scoring elements 34.

The pleating and wrapping arrangement shown in these Figures and disclosed herein is made possible by the use of scoring elements which are rounded at their extremities. More particularly, in the embodiments shown the scoring elements 34 are ribs extending along the length of the balloon and are formed of the same material as the walls 36 of the balloon or of a material which is compatible therewith so as to form a unitary structure with the balloon wall. The scoring elements 34 have an optional neck 35 and a generally triangular or tapering head portion 37 having a rounded extremity 39.

The extremities 39 of the scoring elements 34 are rounded preferably to a radius of at least 0.025 millimetres. Such rounding ensures that the scoring elements 34 do not cut the balloon wall. It has been found that scoring elements 34 of such a nature can be as effective and in some instances more effective than sharp cutting elements in the form of blades. In most instances stenosis material can be removed by scoring rather than cutting the material, and that scoring reduces the risk of damage to the vessel wall.

Preferably, the scoring elements 34 have their extremities 39 rounded to a radius of around 0.025 millimetres to around 0.25 millimetres.

As mentioned above, the scoring elements may be made of a compliant material, in which the extremities of the scoring elements partially flatten or become rounded upon application of pressure thereto, such as not to provide a sharp and hard cutting blade. Flattening of this nature could round the extremities to a radius of at least 0.025 millimetres as disclosed above. Thus, the scoring elements could have sharp extremities when not pressed but in practice do exhibit rounded extremities once pressure is applied to them, thereby avoiding risk of them cutting the balloon wall. Suitable materials for such a balloon includes polyamide such as Nylon™, polyurethane, polyether block amide such as Pebax™ and other materials. Nylon 12™ is preferred.

In the preferred embodiment, the wrapped scoring balloon 50 has minimal airspaces. In particular, the wrapped balloon wall 36 is in direct contact with, that is touches, the scoring elements 34 over which it is wrapped.

The wrapping of the balloon wall 36 is advantageously in the same direction, that is with the sections 38 all being folded clockwise or anti-clockwise.

In an embodiment, the scoring elements 34 are made of a polymeric or elastomeric material. Advantageously, the scoring elements 34 are unitary with the balloon wall 36, that is in the form of a single component. For this purpose, the scoring elements 34 could constitute thickened portions of balloon wall 36 in the form of ribs or ridges. It is preferred that the scoring elements 34 and the balloon wall 36 are made from a single element, typically by extrusion through a suitable dye. For this purpose, the scoring elements are advantageously made of the same material as the balloon wall, such as but not limited to polyurethane, polyethylene terephthalate, polyamide, polyether block amide and so on.

In another embodiment, the scoring elements may be fixed or bonded to the balloon wall, for example by adhesive, weld or bond connections for instance. In this embodiment, the scoring elements could be in the form of a wire or flexible rod attached to the balloon wall so as to be integral therewith. The scoring elements are attached to the balloon wall along their entire lengths.

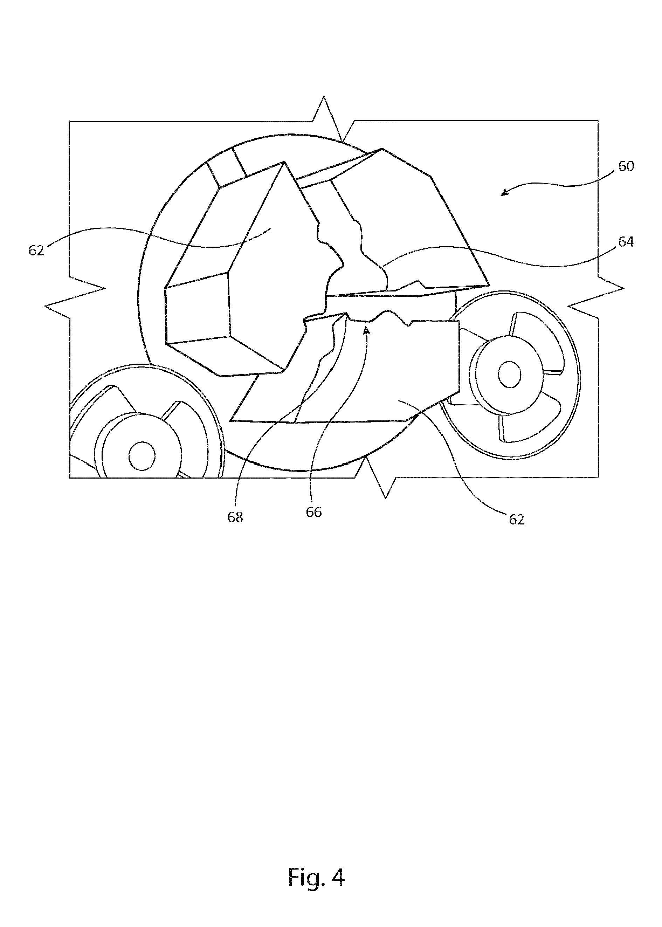

Referring now to Figure 4, there is shown an embodiment of pleating machine and in particular of the pleating blade assembly 60 of such a machine. The pleating blade assembly 60 shown in Figures 4 to 6 has three blades 62 for pleating a balloon 26, 50 having three scoring elements 34. For balloons having a different number of scoring elements, the blade assembly 60 would have a different and equivalent number of pleating blades 62.

Each pleating blade 62 has a length at least as long as the body portion 32 of the balloon 26 and in general will be substantially longer than this so as to be able accommodate different sizes of balloon. Each pleating blade 62 includes an internal rounded surface 64 which extends along its length, as well as a side surface 66 which in the preferred embodiment also has a gentle curvature for facilitating wrapping of the balloon, as will become apparent below.

The edge between the two surfaces 64 and 66 forms a pleating blade element 68 which, as a result of the curvature of surfaces 64 and 66, could be said to be have the form of a bird beak. As will be apparent in Figure 4, the blade elements 68 have a common orientation, which can be described as being clockwise with reference to the view of Figure 4. This common orientation will pleat or fold the balloon 10, 50 into a plurality in this case three, equivalent pleats, all extending in the same rotational direction.

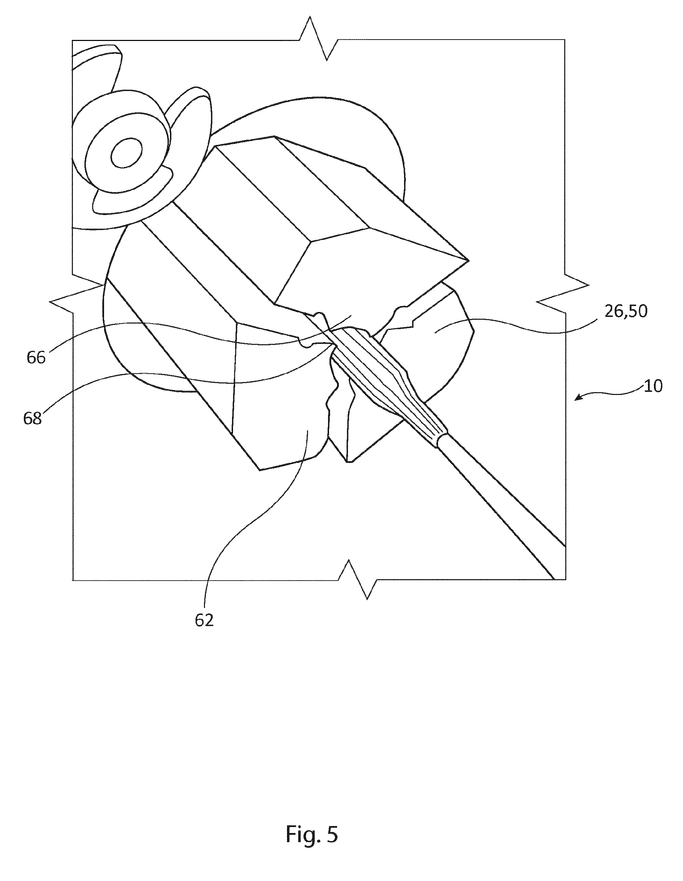

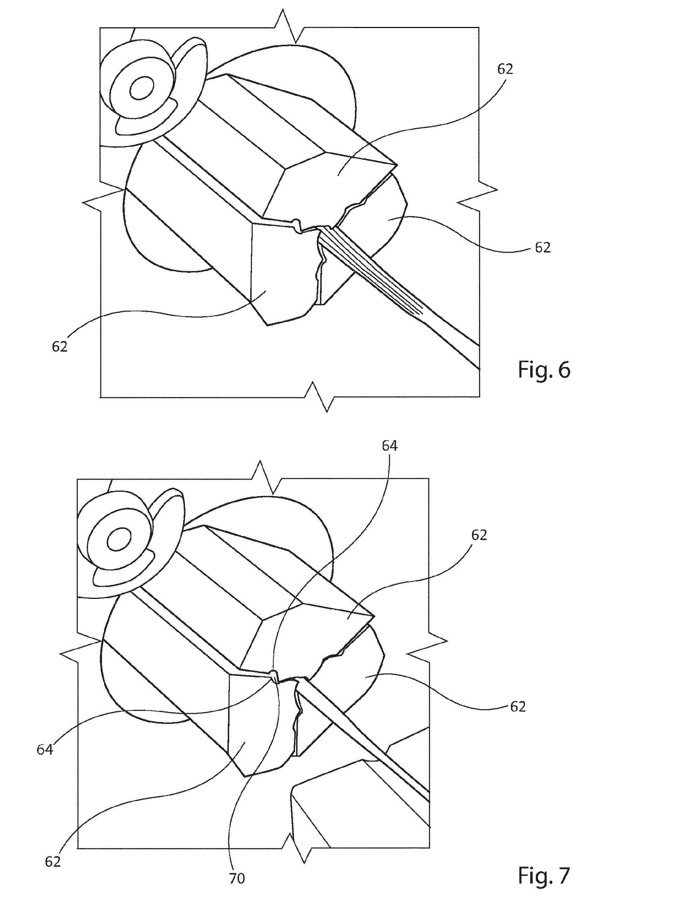

With reference to Figures 5 and 6, a balloon catheter is inserted into the gap between the pleating blades 62. The blades 62 are then gradually brought together so as to close the gap. At the appropriate closing, dependent upon the dimension of the balloon catheter when inserted into the device (at which point the balloon 26, 50 will be in an open configuration), the blade elements 68 will come into contact with the balloon 26,50. More particularly, the blade elements 68 are positioned preferably just beyond a respective scoring element 34, such that the scoring element 34 is on the concave side of each pleating blade 62. As a result, blade elements 68 are able to pleat the balloon wall 36 to overlie the scoring elements 34 as the pleating blades 62 are closed in further. This position of the balloon can be achieved by rotating the balloon catheter 10 until the scoring elements 34 come into position against a respective concave side surfaces 68.

As can be seen in Figure 6, the pleating blades 62 are further closed, thereby causing the balloon wall 36 to be folded over the scoring elements 34.

Figure 7 shows the pleating blades 62 in their fully closed position. It can be seen that the inner surface 64 of one of the pleating blades 62 faces the side surface 66 of the adjacent pleating blade 62. However, there is a gap 70 between these two surfaces 64, 66 which accommodates on of the scoring elements 34. Thus, the pleating blade assembly 60 shown in Figures 4 to 7 is able to pleat balloons having scoring elements 24 already disposed thereon.

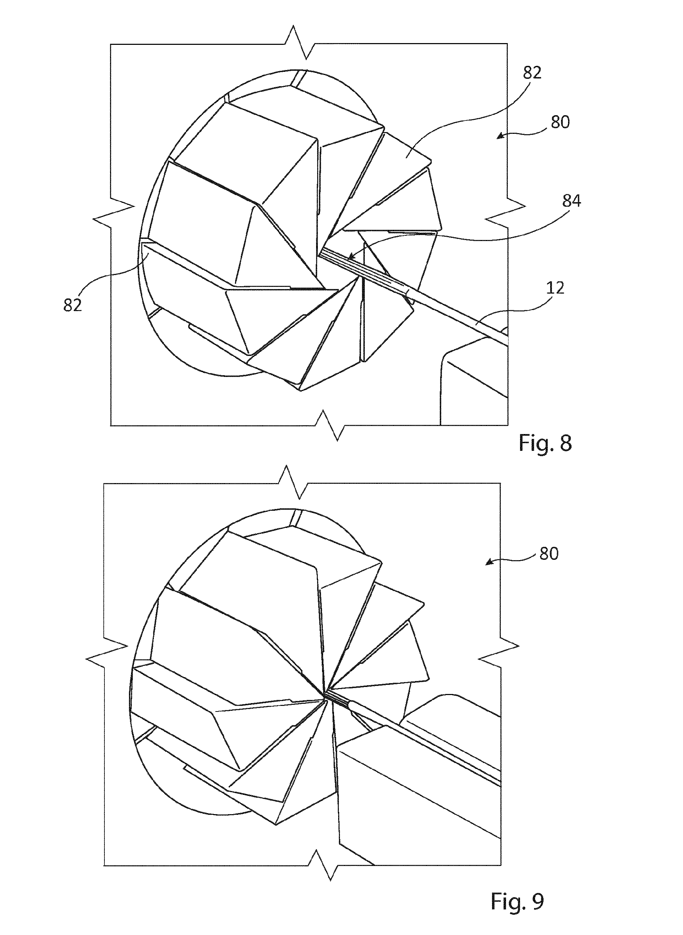

Referring now to Figures 8 and 9, these show a tool 80 for wrapping the pleated balloon. Specifically, after the balloon operation carried out by the pleating blade assembly 60 of Figures 4 to 7, the balloon will typically have a pleated shape as shown in Figure 3. The wrapping tool 80 presses the flaps or wings of the pleated balloon around the catheter 12 so as to form a tightly wrapped structure as shown in Figure 2.

The wrapping tool 80 includes, in this embodiment, a plurality of wedge elements 82 of elongate form which are arranged in an iris configuration able to close in so as to constrict the opening 84 therebetween. This can be seen in particular by a comparison of Figures 8 and 9.

The final wrapped balloon thus has a configuration in which the balloon wall 36 is tightly wrapped over the scoring elements 34, as will be apparent in Figure 2.

Having regard to Figures 4 to 9, it will be apparent that the scoring balloon 10, 50 is inflated so as to attain its open form, then fitted into the pleating blade assembly 60. Once in the assembly 60, the pleating blades 62 are, as necessary, brought close to the outside of the balloon wall 36 and the balloon 10, 50 then rotated so as to position its cutting or scoring elements adjacent the concave side surfaces 68 of the pleating assembly 60. The port 20 is then opened to allow fluid to escape from the balloon, as the folding blades 62 are then moved in. This movement of the pleating blades 62 will pleat the balloon wall 36 over their adjacent scoring elements 34, in the manner shown in Figure 3. Once the pleating blades 62 have been fully closed and the balloon fully pleated, the balloon 10, 50 is withdrawn and then inserted into the wrapping assembly 80. The wedge elements 82 are progressively closed in iris manner, if necessary with suitable rotation of the pleated balloon 10, 50. Once the wrapping wedges 82 have been fully closed, the balloon 10, 50 will have the wrapped configuration shown in Figure 2.

The wrapped balloon 10, 50 will typically be delivered via an introducer sheath of known form, save for the fact that the sheath can have a considerably smaller inner diameter compared to sheaths currently used for delivery of cutting or scoring balloons.

Once delivered and the carried sheath retracted, the balloon 10, 50 is inflated to expand so as to fill the lumen and then rotated and operated in known manner to scrape stenosis material off the vessel wall thereby to open this.

It will be appreciated that described above are preferred embodiments of the present invention and that modifications may be made to these within the scope of the appended claims.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in British patent application number 1217865.3, from which this application claims priority, and in the abstract accompanying this application are incorporated herein by reference.

## Claims

1. A pleated scoring balloon, the balloon including a flexible balloon wall having inner and outer surfaces, the balloon wall providing a balloon body portion extending along a longitudinal axis of the balloon and having a circumferential periphery; and a plurality of scoring elements on or attached to the outer surface of the balloon wall, said scoring elements extending substantially in the direction of said longitudinal axis and being spaced from one another along the circumferential periphery of the body portion, each scoring element exhibiting a rounded or compliant extremity; wherein balloon wall located between the scoring elements overlies and is in contact with the scoring elements.

2. A pleated scoring balloon according to claim 1, wherein the extremities of the scoring elements are rounded to a radius of at least 0.025 millimetres.

3. A pleated scoring balloon according to claim 1 or 2, wherein the scoring elements have their extremities rounded to a radius of 0.025 millimetres to 0.25 millimetres.

4. A pleated scoring balloon according to claim 1, 2 or 3, wherein the balloon is wrapped to have minimal airspaces.

5. A pleated scoring balloon according to any preceding claim, wherein the balloon wall is in direct contact with the scoring elements over which it is wrapped.

6. A pleated scoring balloon according to any preceding claim, wherein the wrapping of the balloon wall is in the same direction.

7. A pleated scoring balloon according to any preceding claim, wherein the scoring elements are made of a polymeric or elastomeric material.

8. A pleated scoring balloon according to any preceding claim, wherein the scoring elements are unitary with the balloon wall.

9. A pleated scoring balloon according to claim 8, wherein the scoring elements constitute thickened portions of balloon wall in the form of ribs or ridges.

10. A pleated scoring balloon according to any preceding claim, wherein the scoring elements are made of the same material as the balloon wall.

11. A pleated scoring balloon according to any preceding claim, wherein the scoring elements are fixed or bonded to the balloon wall.

12. A pleated scoring balloon according to any preceding claim, wherein the scoring elements are in the form of a wire or flexible rod attached to the balloon wall so as to be integral therewith.

13. An introducer assembly including a balloon catheter having a proximal and a distal end and a scoring balloon at the distal end thereof, the scoring balloon being pleated and wrapped on the balloon catheter; the balloon including a flexible balloon wall having inner and outer surfaces, the balloon wall providing a balloon body portion extending along a longitudinal axis of the balloon and having a circumferential periphery; and a plurality of scoring elements on or attached to the outer surface of the balloon wall, said scoring elements extending substantially in the direction of said longitudinal axis and being spaced from one another along the circumferential periphery of the body portion, each scoring element having a rounded or compliant extremity; wherein balloon wall located between the scoring elements overlies and is in contact with the scoring elements.

14. An introducer assembly according to claim 13, wherein the extremities of the scoring elements are rounded to a radius of at least 0.025 millimetres.

15. An introducer assembly according to claim 13 or 14, wherein the scoring elements have their extremities rounded to a radius of 0.025 millimetres to 0.25 millimetres.

16. An introducer assembly according to claim 13, 14 or 15, wherein the wrapped scoring balloon has minimal airspaces.

17. An introducer assembly according to any one of claims 13 to 16, wherein the wrapped balloon wall is in direct contact with the scoring elements over which it is wrapped.

18. An introducer assembly according to any one of claims 13 to 17, wherein the wrapping of the balloon wall is in the same direction.

19. A method of pleating a scoring balloon, the balloon including a flexible balloon wall having inner and outer surfaces, the balloon wall providing a balloon body portion extending along a longitudinal axis of the balloon and having a circumferential periphery; and a plurality of scoring elements on or attached to the outer surface of the balloon wall, said scoring elements extending substantially along said longitudinal axis and being spaced from one another along the circumferential periphery of the body portion; the method including the steps of pleating balloon wall located between the scoring elements so as to overlie the scoring elements and wrapping the balloon wall tightly over the scoring elements.
